# EUROPEAN PATENT SPECIFICATION

(11) **EP 1 277 480 B1**
(45) Date of publication and mention of the grant of the patent: **12.05.2004**
(21) Application number: 00988819.9
(22) Date of filing: 28.12.2000
(51) Int. Cl.: A61L 2/20, A61L 2/26

(54) **APPARATUS FOR DISINFECTING OBJECTS**
VORRICHTUNG ZUM DESINFIZIEREN VON GENGENSTÄNDEN
APPAREIL DE DESINFECTION D'OBJETS

(30) Priority: 30.12.1999 ES 9902880
(43) Date of publication of application: 22.01.2003
(73) Proprietor: Muniain Latasa, Javier Antonio, 31180 Cizur Mayor (ES)
(72) Inventor: Muniain Latasa, Javier Antonio, 31180 Cizur Mayor (ES)
(74) Representative: Evens, Paul Jonathan
(86) International application number: PCT/ES2000/000495
(87) International publication number: WO 2001/049329

(56) References cited:
- WO-A1-99/26668
- ES-B1- 2 116 238
- ES-U- 1 019 087
- FR-A1- 2 351 666
- US-A- 5 520 893

## Description

The object of the present invention refers to an apparatus dedicated to the disinfection of objects of daily use and basically in the domestic environment, with the purpose of avoiding the possible infections that can take place to those who use the aforementioned objects.

Investigations carried out have demonstrated that in the domestic environment a problem exists referring to the disinfection of objects of daily use, to guarantee the absence of noxious microbes that can generate infectious contaminations which can originate several diseases.

At present there are some disinfection methods that solve the problem in connection with certain objects, but in a specific way in each concrete case and not in a general way, also being their use generally uncomfortable and not very practical, so that people end up by not using them, persisting the problem of the disinfection.

The studies carried out have reached the conclusion that the problem of generalizing the disinfection of the domestic objects is conditioned by the shapes of the different objects and the difficulty of being able to reach the critical points where the organisms of difficult elimination get settled down, being it in some cases necessary to use very aggressive methods, which can deteriorate other objects and therefore are impracticable.

ES-2116238 discloses a disinfection support for toothbrushes and comprises a case having a removable container with compartments for the toothbrushes and a housing containing an ozone generator.

US 3,078,526 discloses disinfecting apparatus for shoes or the like, which uses ozone. The apparatus comprises an element placed between the ozone generator and the disinfecting chamber and is provided with two conduits, which define ozone projecting outlets for expelling ozone directly into the objects being disinfected.

To solve the aforementioned problem in an acceptable and practical way, in accordance with the present invention, a disinfection apparatus is proposed which provides a simple comfortable method for the disinfection of any type of object, with very effective results for the mentioned function.

This apparatus object of the invention consists in a container to house the objects to be disinfected, which is coupled in a detachable way with regard to an ozone generator, being included an ozone transmission pipe with multiple projection outlets in the container.

According to the present invention, there is provided an apparatus for disinfecting objects, said apparatus comprising a container to hold the objects, and a housing which includes an ozone generator, the container being incorporated in disassemblable disposition with respect to the housing, characterised in that the container is closable with a cover, the cover having a mounting connection for slidably engaging a guide of the housing, and an element with multiple pipes with projection outlets, the projection outlets being distributed along the inside of the container and configured for projecting ozone directly on the objects.

In one form, an apparatus is provided that allows the ozone disinfection of the critical points of objects that are housed in the container, reaching an appropriate disinfection effectiveness in all the cases; while the inside of the container is shaped to favour the generation of turbulence in the projected ozone, providing in turn a great effectiveness in the perimetric disinfection of the objects.

The ozone generator may consist of a receptacle in which lamps are housed which transform O₂ oxygen into O₃ ozone, the receptacle having an air inlet provided with a filter and at the opposite end an outlet that connects by means of a piping with the ozonised air transmission means to the container with the objects to be disinfected, in connection with whose ozonised air transmission piping a driving compressor is included.

The container for the objects may be provided with a cover which has an adjustable opening that is foreseen of a grip to ease up the manipulation, being included in connection with this cover a safety lock that determines the automatic disconnection of the operation of the apparatus while the object container is not perfectly closed.

In view of all this, the mentioned object disinfecting apparatus of the invention certainly has some very advantageous characteristics, acquiring own life and preferable character, regarding the conventional methods, for the disinfecting function to which it is dedicated.

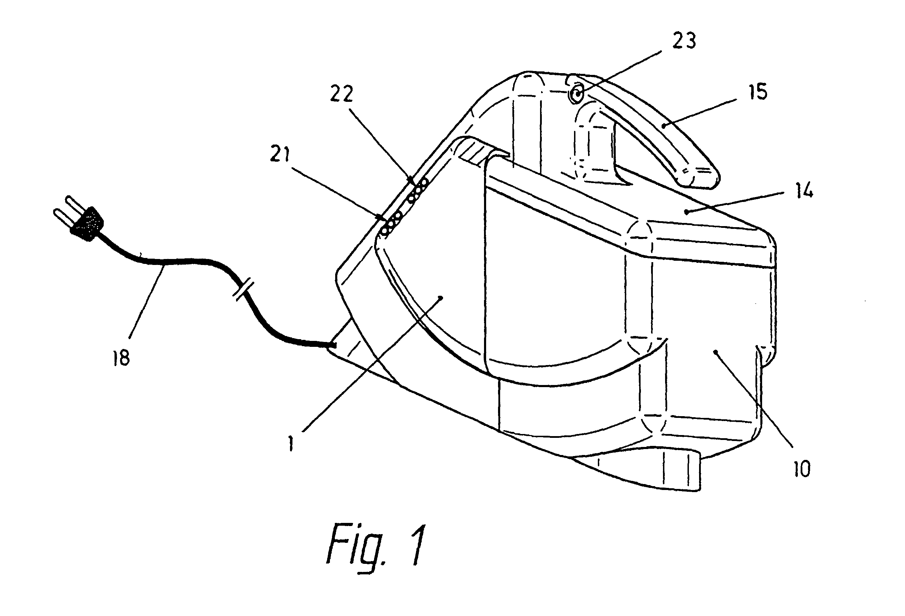

Figure 1 shows a perspective of the preconized disinfecting apparatus, according to a concrete realization example.

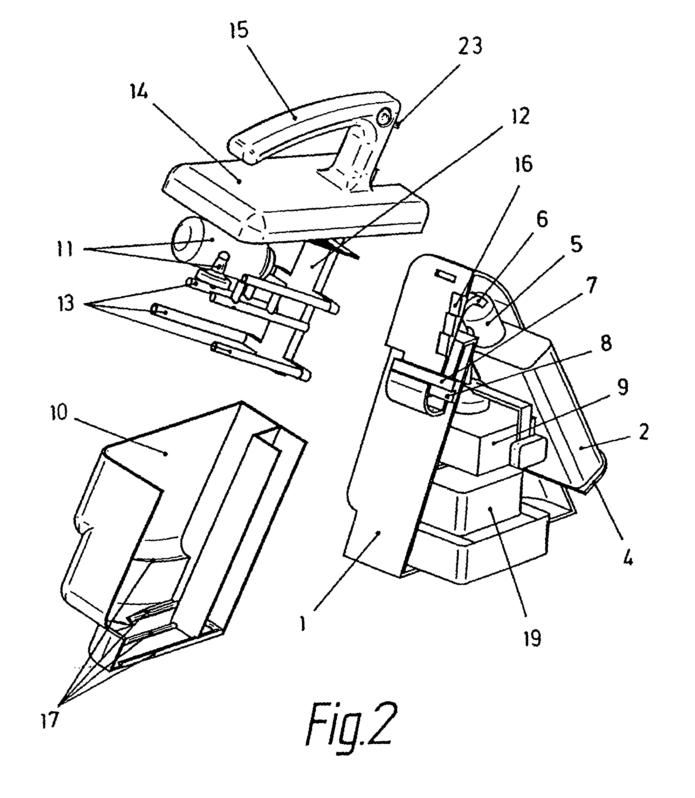

Figure 2 is an exploded perspective of the disinfecting apparatus with the object container and the housing corresponding to the ozone generating group.

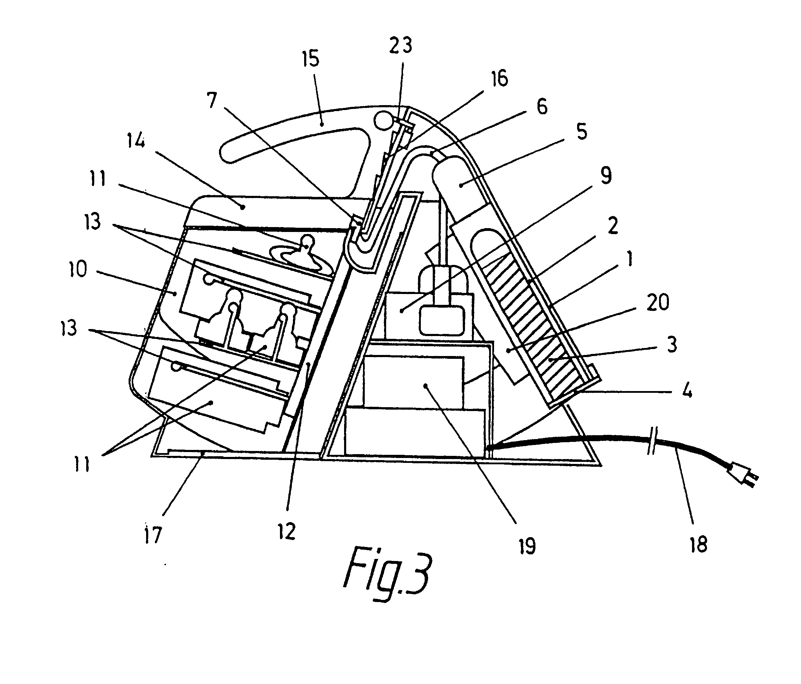

Figure 3 is a sectioned side view of the mounted disinfecting group.

The object of the invention refers to an apparatus dedicated to the disinfection of objects of daily use in the domestic environment, being effective for the disinfection of different types of objects, without their shape being an impediment.

The mentioned apparatus includes a functional group made up of a housing (1) in whose inside a receptacle (2) is housed which contains one or multiple lamps (3) which transform O₂ oxygen into O₃ ozone.

The aforementioned receptacle (2) has an air inlet, in which a filter (4) is incorporated to avoid the passage of dirt, remaining defined at the opposite end an outlet (5) connected to a pipe (6) that ends up in a diffuser (7), which incorporates an anti-return membrane (8) to avoid the recession.

Inside the housing (1) a compressor (9) is moreover included which is related to the outlet (5), to produce an impulsion through the pipe (6) towards the diffuser (7).

The apparatus includes moreover a container (10), dedicated to house in its inside the objects (11) to be disinfected, which can be of any kind, and of which the disinfection results useful or necessary, such as baby bottle teats, containers, toothbrushes, etc.

The mentioned container (10) can be connected in a detachable way with regard to the functional group, including an element (12) between both which includes multiple pipes (13) determining projection outlets which remain distributed by the inside of the container (10).

That element (12) bearer of the pipes (13) with the projection outlets, is associated to a cover (14), by means of which the container (10) is closed, having a grip (15) which eases up the manipulation, as well as the transport of the set together with the apparatus.

The cover (14) can be connected in guided assembly, passing through a guide (16) which allows the selective opening and closure, so that in closed disposition, the element (12) out of which the pipes (13) leave remain related to the outlet of the pipe (6), communicating the latter with the projection outlets distributed along the container inside (10).

With all this, the air which enters the receptacle (2) through the filter (4), is ozonized by the lamps (3), passing the ozonized air through the pipe (6), thanks to the impulsion originated by the compressor (9), so that at the outlet of the mentioned pipe (6) the ozonized air is projected through the diffuser (7), passing through the element (12) to the pipes (13), to leave projected through the outlets determined inside the container (10).

This way, the ozonized air is projected directly on the crevices of the objects (11) to be disinfected, reaching efficiently all their critical points.

On the other hand, inside the container (10) some conformations (17) are foreseen in the form of entrances and outlets, thanks to which a cyclic turbulence of the ozonized air, which is projected inside the mentioned container (10) is reached, obtaining a distribution of the flow which makes the perimetric disinfection of the objects (11) of application completely effective.

The operation of the apparatus is electric, being equipped with a connection cable (18) for the electric supply from the feeding network, including inside the housing (1) a transformer (19) for the electric supply to the operation equipment, while by means of an electronic board (20) the needed regulation is reached, the control of the push-buttons (21) to control the device, the regulation of the established disinfection times and also the control, of the luminous signalling devices (22) which indicate the state of the device in the disinfection process.

In connection with the closing cover (14) of the container (10) a security closure (23) is installed, which makes up the electric switch, determining the automatic disconnection of the operation when it is opened and it does not allow the activation of the operation while the container (10) is not completely closed.

## Claims

1. An apparatus for disinfecting objects, said apparatus comprising a container (10) to hold the objects (11), and a housing (1) which includes, an ozone generator (3, 9) the container (10) being incorporated in disassemblable disposition with respect to the housing (1), **characterised in that** the container (10) is closable with a cover (14), the cover having a mounting connection for slidably engaging a guide (16) of the housing (1), and an element (12) with multiple pipes (13) with projection outlets, the projection outlets being distributed along the inside of the container and configured for projecting ozone directly on the objects.

2. An apparatus according to the first claim, in which a receptacle (2), which includes the ozone generator (3), is situated in the housing (1), the receptacle having an air entrance (4) at a first end and an outlet (5) at a second opposite end that is connected by a pipe (6) to the element (12) when the container (10) is closed by the cover (14).

3. An apparatus according to the first claim, in which the inside of the container (10) defines conformations (17) in the form of cavities and projections, which generate a turbulence of the projected ozone flow for perimetric disinfection of the objects (11).

4. An apparatus according to the first claim, in which in connection with the cover (14) for closing the container (10), a security lock (23) is situated, which acts as an electric switch configured to be disconnected and prevent operation while the container (10) is not perfectly closed.

## Patentansprüche

1. Vorrichtung zur Desinfektion von Objekten, die umfasst: einen Behälter (10) zur Aufnahme der Objekte (11) und ein Gehäuse (1), das einen Ozongenerator (3, 9) einschließt, wobei der Behälter (10) in einem demontierbaren Zustand bezogen auf das Gehäuse (1) inkorporiert ist, **dadurch gekennzeichnet, dass** der Behälter (10) mit einem Deckel (14) verschließbar ist, wobei der Deckel ein Montageverbindungsstück aufweist, das durch Schieben mit einer Führung (16) des Gehäuses (1) in Eingriff gebracht wird, und ein Element (12) mit einer Mehrzahl von Röhren (13) mit Ausstoßöffnungen aufweist, wobei die Ausstoßöffnungen entlang der Innenseite des Behälters verteilt sind und so gestaltet sind, dass sie Ozon so ausstoßen, das dieses direkt auf die Objekte trifft.

2. Vorrichtung nach Anspruch 1, wobei ein Behältnis (2), das den Ozongenerator (3) einschließt, in dem Gehäuse (1) angeordnet ist, wobei das Behältnis an einem ersten Ende einen Lufteinlass (4) aufweist und an einem zweiten, gegenüberliegenden Ende einen Auslass (5) aufweist, der durch eine Röhre (6) mit dem Element (12) verbunden ist, wenn der Behälter (10) durch den Deckel (14) verschlossen ist.

3. Vorrichtung nach Anspruch 1, wobei die Innenseite des Behälters (10) Gebilde (17) in Form von Vertiefungen und Erhöhungen definiert, die eine Verwirbelung des ausgestoßenen Ozonstroms zwecks der umfänglichen Desinfektion der Objekte (11) bewirken.

4. Vorrichtung nach Anspruch 1, wobei in Zusammenhang mit dem Deckel (14) zum Schließen des Behälters (10) ein Sicherheitsverschluss (23) angeordnet ist, der als elektrischer Schalter fungiert, der so gestaltet ist, dass er unterbrochen ist und einen Betrieb verhindert, solange der Behälter (10) nicht ganz geschlossen ist.

## Revendications

1. Appareil pour désinfecter des objets, ledit appareil comprenant un récipient (10) pour contenir les objets (11), et un logement (1) qui comprend un générateur d'ozone (3, 9), le récipient (10) étant incorporé dans une disposition pour être désassemblé par rapport au logement (1), **caractérisé en ce que** le récipient (10) peut être fermé avec un couvercle (14), le couvercle ayant une liaison de montage pour venir en prise de façon coulissante avec un guide (16) du logement (1), et un élément (12) avec de multiples tuyaux (13) avec des sorties en saillie, les sorties en saillie étant distribuées le long de l'intérieur du récipient et configurées pour projeter de l'ozone directement sur les objets.

2. Appareil selon la première revendication, dans lequel un réceptacle (2), qui comprend le générateur d'ozone (3), est situé dans le logement (1), le réceptacle ayant une entrée d'air (4) au niveau d'une première extrémité et une sortie (5) au niveau d'une seconde extrémité opposée qui est raccordée par un tuyau (6) à l'élément (12) lorsque le récipient (10) est fermé par le couvercle (14).

3. Appareil selon la première revendication, dans lequel l'intérieur du récipient (10) définit des conformations (17) ayant la forme de cavités et saillies, qui génèrent une turbulence de l'écoulement d'ozone projeté pour une désinfection périmétrique des objets (11).

4. Appareil selon la première revendication, dans lequel conjointement avec le couvercle (14) pour fermer le récipient (10), un dispositif de verrouillage (23) est situé, qui agit en tant que commutateur électrique configuré pour être déconnecté et empêcher le fonctionnement lorsque le récipient (10) n'est pas parfaitement fermé.
